# EUROPÄISCHE PATENTANMELDUNG

(11) **EP 1 300 385 A1**
(43) Veröffentlichungstag der Anmeldung: **09.04.2003**
(21) Anmeldenummer: 02019872.7
(22) Anmeldetag: 10.09.2002
(51) Int. Cl.: C07C 51/09, C07C 57/10

(54) **Verfahren zur Herstellung von Sorbinsäure aus Sorbinsäurepolyester**

(30) Priorität: 21.09.2001 DE 10146689
(71) Anmelder: Nutrinova Nutrition Specialties & Food Ingredients GmbH, 65929 Frankfurt am Main (DE)
(72) Erfinder: Mollenkopf, Christoph Dr., 65929 Frankfurt (DE); Weiss, Erwin Dr., 65719 Hofheim (DE); Beck, Thomas Dr., 65510 Idstein (DE); Schneider, Andreas, 61462 Königstein (DE); Fahrner, Klaus, 65774 Kelkheim (DE); Purps, Stefan, 61462 Königstein (DE)
(74) Vertreter: Schweitzer, Klaus, Dr.

(57) **Zusammenfassung**

Die Erfindung betrifft ein Verfahren zur Herstellung von Sorbinsäure durch Spaltung des aus Crotonaldehyd und Keten hergestellten Sorbinsäurepolyesters, wobei der Sorbinsäurepolyester destilliert wird und wobei die Spaltung durch ein Amin katalysiert wird, dadurch gekennzeichnet, dass das Amin aus dem Destillationsrückstand durch Destillation unter reduziertem Druck (und bei einer Temperatur, die höher ist als die Temperatur der Polyesterdestillation) abgetrennt und zurückgewonnen wird.

## Beschreibung

Zur Herstellung von Sorbinsäure sind verschiedene Verfahren bekannt. Ein besonders wirtschaftliches Verfahren geht von dem polymeren Reaktionsprodukt Polyester aus, der durch Umsetzung von Crotonaldehyd mit Keten in einem inerten Lösungsmittel in Gegenwart eines fettsauren Salzes eines zwei- und/oder dreiwertigen Metalls der II. bis VIII. Nebengruppe des Periodensystems als Katalysator hergestellt wird **(DE-A-10 42 573).**

Aus diesem Polyester kann auf verschiedenen Wegen Sorbinsäure gewonnen werden.

Ein technisch bedeutsames Verfahren besteht aus der thermisch katalytischen Spaltung des Polyesters, das dadurch gekennzeichnet ist, dass man die Spaltung des Polyesters in Gegenwart eines inerten und unter Normaldruck über 150 °C, vorzugsweise über 180 °C, siedenden Verdünnungsmittel **(DE-A-10 59 899)** und von 0,5% bis 50 % eines sekundären oder tertiären, unter Normaldruck oberhalb 100 °C, vorzugsweise oberhalb 150 °C, siedenden Amins als Katalysator bei Temperaturen von 160 °C bis 220 °C unter gleichzeitigem Abdestillieren der Sorbinsäure und des Verdünnungsmittels durchführt **(DE-A-12 82 645)**. Besonders geeignet als Lösungsmittel sind die in DE-A-10 59 899 genannten aliphatischen Carbonsäuren mit entsprechendem Siedepunkt.

Bei der technischen Durchführung dieses Verfahrens wird die Spaltung in einer kontinuierlich betriebenen Destillationsapparatur durchgeführt. Der in dem Verdünnungsmittel gelöste Sorbinsäurepolyester wird in die Destillationsblase eindosiert, wo die aminkatalysierte Spaltung des Sorbinsäurepolyesters zur Sorbinsäure stattfindet. Die gebildete Sorbinsäure wird zusammen mit dem Verdünnungsmittel über eine Rektifikationskolonne bei 160 - 200 °C und 20 - 50 HPa mit Rücklauf abdestilliert. Die Rektifikation ist notwendig, um den Übergang des Amins in das Destillat zu verhindern und zur Erzielung der entsprechenden Reinheit.

Aus dem Destillat wird anschließend die Sorbinsäure auskristallisiert und von dem Verdünnungsmittel abgetrennt. Das Verdünnungsmittel wird im Kreis geführt.

Parallel zu der gewünschten Spaltung des Sorbinsäurepolyesters zu Sorbinsäure findet eine Decarboxylierungsreaktion des Sorbinsäurepolyesters zu Kohlendioxid und Pentadien statt und mindert so die Ausbeute. Diese Reaktion kann durch Erhöhung des Amingehaltes im Sumpf der Spaltung zum größten Teil unterdrückt werden. So erhält man zum Beispiel eine ca. 4 % höhere Ausbeute an Sorbinsäure, wenn die Aminkonzentration im Sumpf der Spaltung von 10 % auf 40 % angehoben wird.

Da bei der thermischen Sorbinsäurepolyesterspaltung in der Destillationsblase neben der Sorbinsäure auch Polymere entsteht, die unter diesen Bedingungen nicht destillieren und zu einem Sumpfzuwachs führen, müssen diese kontinuierlich gebildeten Polymere ständig als Rückstand ausgeschleust werden. Da das Katalysatoramin mit den Polymeren vermischt ist, wird unvermeidlich damit auch Katalysatoramin aus dem Sumpf mitausgeschleust, so dass zur Erhaltung der Aminkonzentration im Sumpf der Spaltung ständig neues Amin zugesetzt werden muß.

Um zur Realisierung der Ausbeuteerhöhung der Sorbinsäurepolyesterspaltung die Konzentration des Katalysatoramins im Sumpf zu vervierfachen, muß der Amineinsatz ebenfalls ungefähr vervierfacht werden. Da dieses Amin mit dem Polymerisat aus dem Sumpf wieder ausgeschleust werden muß, bedeutet dies aber gleichzeitig auch eine Vervielfachung der Rückstandsmenge an Amin.

Es bestand daher die Aufgabe, ein Verfahren zu entwickeln, bei dem das Katalysatoramin aus dem Rückstand wiedergewonnen wird und damit zum erneuten Einsatz als Katalysator zur Sorbinsäurepolyesterspaltung wieder eingesetzt werden kann.

Die Erfindung betrifft daher ein Verfahren zur Herstellung von Sorbinsäure durch thermische Spaltung des aus Crotonaldehyd und Keten hergestellten Polyesters, bei dem aus dem zwangsläufig anfallenden Rückstand das als Katalysator eingesetzte Amin zurückgewonnen wird und somit zum Wiedereinsatz kommen kann. Dadurch eröffnet sich zum einen die Möglichkeit, die Aminkonzentration im Sumpf der Spaltung zur Erzielung einer höheren Ausbeute an Sorbinsäure zu erhöhen, ohne dass Amin verloren geht. Zum anderen kann dadurch der Einsatz frischen Amins reduziert werden. Zusätzlich geht damit eine Reduktion der Gesamtrückstandsmenge einher.

Überraschenderweise wurde gefunden, dass durch eine Destillation des Rückstands bei 190 bis 220 °C, bevorzugt 205 bis 215 °C und einem Druck von 5 bis 15 HPa, bevorzugt 7 bis 9 HPa selektiv das Katalysatoramin von den sonstigen Sumpfbestandteilen abgetrennt werden kann.

Vorteilhaft läßt sich das Verfahren in einem Dünnschichtverdampfer durchführen. Besonders gute Ergebnisse erhält man bei Verwendung eines tertiären Amins als Katalysator, insbesondere einem Trialkylamin mit zwei C₁-C₃-Alkylgruppen, insbesondere Methylgruppen und einer Alkylkette mit 14 bis 20 Kohlenstoffatomen, insbesondere 15 bis 17, ganz besonders bevorzugt 16 Kohlenstoffatomen.

Zur Durchführung der Spaltung des Sorbinsäurepolyesters sind als Verdünnungsmittel aliphatische, alicyclische, aromatische Kohlenwasserstoffe, deren Chlor-, Brom- und Nitroderivate sowie auch Ether und Siliconöle geeignet, deren Siedepunkt bei normalem Druck über 150 °C, vorzugsweise über 180 °C liegen. Aber auch Ketone, Ester, Carbonsäuren und Alkohole mit dem entsprechenden Siedebereich können als Verdünnungsmittel herangezogen werden, obwohl im allgemeinen die Ergebnisse nicht ganz so gut sind, da sie sich offenbar teilweise mit dem Reaktionsgemisch umsetzen. Es ist zweckmäßig, solche Verdünnungsmittel bzw. Lösungsmittel zu verwenden, die bei normalen Temperaturen flüssig sind, bei normalem Druck unter 300 °C, vorzugsweise unter 270 °C sieden und mit der Sorbinsäure azeotrope Gemische bilden, so dass sie auch gleichzeitig als Träger- oder Schleppmittel dienen, wie Petroliumfraktionen, Dodekan, Tetradekan, 5-Methyldodekan, Dodecen, Dicyclohexylmethan, p-Di-tertiär-butylbenzol, 1-Methylnaphthalin, 2-Methylnaphthalin, 1-Ethylnaphthalin, Tetrahydronaphthalin, Diphenylnaphthalin; halogenierte aliphatische, cycloaliphatische oder aromatische Kohlenwasserstoffe wie Dichlordodekan, 1,5-Dibrompentan, Benzotrichlorid, o- und m-Dibrombenzol; Nitroverbindungen wie Nitrobenzol, 2-Nitrotoluol; Nitrile wie Benzylcyanid; Carbonylverbindungen wie Acetophenon oder das heterocyclische 2-Acetylthiophen; heterocyclische Verbindungen wie Chroman, Thiophen; Ether wie Resorcindimethylether, Diphenylether, Safrol, Isosafrol; Säuren wie Önanthsäure, α-Ethylcapronsäure, Caprylsäure, Caprinsäure; oder Ester wie Benzoesäureethylester, Phenylessigsäuremethylester und Salicylsäuremethylester.

Die folgenden Beispiele erläutern die Erfindung.

Als Ausgangsmaterial dient ein polyesterhaltiges Umsetzungsprodukt, das analog der **DE-B-10 42 573**, Beispiel 1, erhalten wurde. Dabei werden in ein gerührtes Gemisch aus 800 g Crotonaldehyd, 1200 ml Toluol und 14,2 g Zinkisovalerianat bei einer Temperatur zwischen 25 °C und 35 °C 420 g Keten eingeleitet. Der Überschuß an Crotonaldehyd und das Toluol werden im Vakuum entfernt. Als Rückstand werden 1150 g Polyester in Form einer hochviskosen, braungefärbten Flüssigkeit erhalten. Neben dem Zinkgehalt von 3000 ppm enthält dieses Reaktionsprodukt noch Anteile, die nicht in Hexadiensäuren umgewandelt werden können, wie Diketenpolymerisate und Crotonaldehydharze.

Der in Hexadiensäuren umwandelbare Anteil wurde bestimmt durch basische Verseifung einer Lösung aus 60 g Sorbinsäurepolyester in 120 g Toluol mit 33 g Kaliumhydroxid in 260 g Wasser bei Raumtemperatur. Dabei erhält man in der wäßrigen Phase Kaliumsorbat und das Kaliumsalz der 3-Hydroxy-4-hexensäure, aus denen Hexadiensäure durch Ansäuern gewonnen werden kann. Durch quantitative Bestimmung der beiden Reaktionsprodukte mittels HPLC kann der in Hexadiensäuren unwandelbare Anteil des Polyesters bestimmt werden.

Durch diese milden Bedingungen läßt sich der Polyestergehalt sehr viel genauer als in **DE-A-12 82 645** beschrieben, bestimmen. So beträgt der zu Hexadiensäuren umwandelbare Anteil des Rohpolyesters 89 bis 90 % und nicht, wie in **DE-A-12 82 645** angenommen, nur 80 %. Die in **DE-A-12 82 645** erzielten Ausbeuten müssen daher, siehe Beispiel 1 (Vergleichsbeispiel), korrigiert werden.

### Beispiel 1 (Vergleichsbeispiel)

Die Apparatur besteht aus einem 1I - 3H-Rundkolben (Reaktionskolben) mit aufgesetzter Destillationskolonne. Die Destillationskolonne mit einer Füllhöhe von 600 mm und einem Innendurchmesser von 40 mm ist mit Raschigringen aus Glas von 6 mm Durchmesser gefüllt. Die Destillationskolonne trägt einen auf 70 °C gekühlten Kolonnenkopf mit Rücklaufteiler. Das kondensierte Destillat wird durch den Rücklaufteiler zum einen in die Kolonne zurückgeführt, zum anderen in einer graduierten, beheizbaren Vorlage (500 ml) und einem 6 I Rundkolben aufgefangen. Die gesamte Apparatur wird unter Vakuum betrieben, zur Vakuumerzeugung dient eine Ölpumpe mit vorgeschalteter Trockeneiskühlfalle.

Diese Apparatur wird semibatchweise betrieben, wobei zur Erlangung einer statistisch abgesicherten Aussagekraft der Ergebnisse mehrere Versuche unter Wiedereinsatz des im jeweiligen Vorversuch erhaltenen Filtrats und Sumpfs durchgeführt wird.

Beim 1. Versuch werden im Reaktionskolben 260 g einer Mischung bestehend aus 12 % Dimethylhexadecylamin und 88 % Arkopal® (= Nonylphenol Polyglykolether als Rückstandsverflüssiger) vorgelegt.

Die Apparatur wird auf etwa 30 HPa evakuiert und der Reaktionskolben mit dem Ölbad (Badtemperatur ca. 220°C) angeheizt. Erreicht die Temperatur im Reaktionskolben 180 °C wird mit der Dosierung (417 g/h) des Einsatzgemisches in den Reaktionskolben bei einem Rücklaufverhältnis von 1 begonnen.

Das Einsatzgemisch für den Reaktionskolben besteht aus 350 g Polyester (siehe oben), 2128 g Ethylhexansäure, 12 g Dimethylhexadecylamin und 10 g Arkopal® (= Nonylphenol Polyglykolether als Rückstandsverflüssiger) ( Gesamtmenge 2500 g).

Nach der Dosierung des Einsatzgemisches wird für 2 Stunden reine 2-Ethylhexansäure ohne Polyester und ohne Rücklauf durch die Apparatur gefahren (834 g) und danach 5 min nachdestilliert. Das in der Vorlage befindliche Destillat wird durch Erwärmen auf 50 - 55°C homogenisiert und anschließend unter Rühren (500 min⁻¹) innerhalb von 3 Stunden auf 20°C abgekühlt. Nach Erreichen dieser Temperatur wird noch 15 min bei 20°C gehalten und danach die kristallisierte Rohsorbinsäure abgesaugt und der Reingehalt gaschromatographisch bestimmt.

Bei diesem Versuch wächst der Sumpf im Reaktionskolben um 62 g an (ausgehend von 260 g). Dieser Rückstandszuwachs besteht aus 12 g Dimethylhexadecylamin, 10 g Arkopal, 2g Sorbinsäure und 2-Ethylhexansäure und 38 g Sorbinsäurepolymere und wird aus dem System ausgeschleust.

Bei jedem weiteren Versuch wird nach der Abtrennung der Rohsorbinsäure das Filtrat anstelle der reinen 2-Ethylhexansäure zusammen mit 350 g Polyester, 12 g Dimethylhexadecylamin und 10 g Arkopal im Einsatzgemisch eingesetzt.

Im Reaktionskolben wird jetzt 260 g des Sumpfes aus dem jeweiligen Vorversuch vorgelegt, der eine durchschnittliche Konzentration an Dimethylhexadecylamin von 12% hat.

Nach mehreren Versuchsdurchführungen erhält man eine durchschnittliche Ausbeute an Sorbinsäure von 74 %. Bezogen auf den Reinpolyester, also unter alleiniger Berücksichtigung des zu Hexadiensäuren spaltbaren Anteils von 90 %, errechnet sich damit eine Ausbeute von 82,2 %.

### Beispiel 2

Die Durchführung der Sorbinsäurepolyesterspaltung erfolgt wie im Beispiel 1 (Vergleichsbeispiel). Beim 1. Versuch werden im Reaktionskolben 260 g einer Mischung bestehend aus 40 % Dimethylhexadecylamin und 60 % Arkopal® (=Nonylphenol Polyglykolether als Rückstandsverflüssiger) vorgelegt.

Das Einsatzgemisch für den Reaktionskolben besteht aus 350 g Polyester (aus Beispiel 1), 2128 g 2-Ethylhexansäure, 48 g Dimethylhexadecylamin und 14 g Arkopal (Gesamtmenge 2540 g).

Der Sumpf bei dieser Reaktion wächst um 104 g an. Dieser Rückstandszuwachs besteht aus 48 g Dimethylhexadecylamin, 14 g Arkopal, 4 g Sorbinsäure und Verdünnungsmittel und 38 g Sorbinsäurepolymere und muß aus dem System vor der nächsten Versuchsdurchführung ausgeschleust werden.

Das Filtrat wird nach der Abtrennung der Rohsorbinsäure im folgenden Versuch wieder anstelle der 2-Ethylhexansäure zusammen mit 350 g Polyester, 48 g Dimethylhexadecylamin und 12 g Arkopal im Einsatzgemisch eingesetzt.

Im Reaktionskolben wird jetzt 260 g des Sumpfes aus dem jeweiligen Vorversuch vorgelegt, der eine durchschnittliche Konzentration an Dimethylhexadecylamin von 40 % hat.

Nach mehreren Versuchsdurchführungen erhält man eine durchschnittliche Ausbeute an Sorbinsäure von 79,9 %. Bezogen auf den Reinpolyester, also unter alleiniger Berücksichtigung des zu Hexadiensäuren spaltbaren Anteils von 90 %, errechnet sich damit ein Ausbeute von 88,7 %.

### Beispiel 3

Die Durchführung der Sorbinsäurepolyesterspaltung erfolgt wie im Beispiel 2.

Wie im Beispiel 2 wächst der Sumpf bei dieser Reaktion um 104 g an. Dieser Rückstandszuwachs besteht aus 48 g Dimethylhexadecylamin, 14 g Arkopal, 4 g Sorbinsäure und 2-Ethylhexansäure und 38 g Sorbinsäurepolymere und muß aus dem System vor der nächsten Versuchsdurchführung ausgeschleust werden.

In einem Dünnschichtverdampfer wird dieser ausgeschleuste Sumpfrückstand bei 210 °C / 8 HPa destilliert. Bei einer Heizfläche des Dünnschichtverdampfers von 16 cm² ist ein Durchsatz von 450 g/h möglich. Der Rotor mit beweglichen Wischerblättern hat eine Umlaufgeschwindigkeit von 3 m/s. Es entstehen 205 g/h Destillat und 245 g/h Rückstandsablauf. Bezogen auf den Einsatz von 104 g erhält man 47 g Destillat und 57 g Rückstand.

Im Dünnschichtverdampferablauf befinden sich die Sorbinsäurepolymere und der Verflüssiger Arkopal neben geringen Mengen Dimethylhexadecylamin. Im Destillat erhält man den größten Teil des ausgeschleusten Amins (43 g) und 4 g Sorbinsäure bzw. 2-Ethylhexansäure.

Dieses Destillat wird mit 5 g frischem Dimethylhexadecylamin auf 48 g Gesamtamin ergänzt und im nächsten Spaltversuch zusammen mit dem Verdünnungsmittel aus der Rohsorbinsäureabtrennung, 350 g Polyester und 12 g Arkopal analog zum Beispiel 2 wieder eingesetzt.

Nach mehreren Versuchsdurchführungen unter Kreisführung des Amins erhält man eine durchschnittliche Ausbeute an Sorbinsäure von 79,9 %. Bezogen auf den Reinpolyester, also unter alleiniger Berücksichtigung des zu Hexadiensäuren spaltbaren Anteils von 90 %, errechnet sich damit eine Ausbeute von 88,7 %.

Zusammengefaßt sind in der folgenden Tabelle Ausbeuten und Amineinsatz der jeweiligen Versuche gegenübergestellt:

| | Beispiel 1 | Beispiel 2 | Beispiel 3 |
|---|---|---|---|
| Amingehalt im Sumpf | 12% | 40% | 40% |
| Aminrückführung | nein | nein | ja |
| Ausbeute | 82,2% | 88,7% | 88,7 % |
| Rückstandszuwachs während der Reaktion | 62g | 104g | 104 g |
| Amineinsatz "neu" | 12g | 48g | 5 g |
| Amineinsatz aus Aminrückführung | | | 43 g |
| aus dem System ausgeschleuster Rückstand | 62 g | 104 g | 57 g |

## Patentansprüche

1. Verfahren zur Herstellung von Sorbinsäure durch Spaltung des aus Crotonaldehyd und Keten hergestellten Sorbinsäurepolyesters, wobei der Sorbinsäurepolyester thermisch gespalten wird und die Spaltprodukte destilliert werden und wobei die Spaltung durch ein Amin katalysiert wird, **dadurch gekennzeichnet, daß** das Amin aus dem Destillationsrückstand durch Destillation unter reduziertem Druck (und bei einer Temperatur, die höher ist als die Temperatur der Polyesterdestillation) abgetrennt und zurückgewonnen wird.

2. Verfahren nach Anspruch 1, **dadurch gekennzeichnet, daß** die Destillation der Spaltprodukte in Gegenwart eines Verdünnungsmittels durchgeführt wird.

3. Verfahren nach Anspruch 2, **dadurch gekennzeichnet, daß** das Verdünnungsmittel ausgewählt wird aus aliphatischen, alicyclischen, aromatischen Kohlenwasserstoffe, deren Chlor-, Brom- und Nitroderivaten sowie auch Ether und Siliconöle.

4. Verfahren nach einem oder mehreren der Ansprüche 1 bis 3, **dadurch gekennzeichnet, daß** die Destillation der Spaltprodukte unter reduziertem Druck bei 20 bis 50 HPa durchgeführt wird.

5. Verfahren nach einem oder mehreren der Ansprüche 1 bis 4, **dadurch gekennzeichnet, daß** die Destillation der Spaltprodukte unter reduziertem Druck bei 160 bis 200 °C durchgeführt wird.

6. Verfahren nach einem oder mehreren der Ansprüche 1 bis 5, **dadurch gekennzeichnet, daß** das Amin ausgewählt wird aus der Gruppe der sekundären oder tertiärem Aminen mit einem Siedepunkt-bei Normaldruck oberhalb von 100 °C vorzugsweise oberhalb von 150 °C.

7. Verfahren nach einem oder mehreren der Ansprüche 1 bis 6, **dadurch gekennzeichnet, daß** die Destillation des Destillationsrückstandes unter reduziertem Druck in einem Dünnschichtverdampfer durchgeführt wird.

8. Verfahren nach einem oder mehreren der Ansprüche 1 bis 7, **dadurch gekennzeichnet, daß** die Destillation des Destillationsrückstandes unter reduziertem Druck bei 5 - 15 HPa in einem Dünnschichtverdampfer durchgeführt wird.

9. Verfahren nach einem oder mehreren der Ansprüche 1 bis 8, **dadurch gekennzeichnet, daß** die Destillation des Destillationsrückstandes unter reduziertem Druck und einer Temperatur von 190 - 220 °C in einem Dünnschichtverdampfer durchgeführt wird.

10. Verfahren nach einem oder mehreren der Ansprüche 1 bis 9, **dadurch gekennzeichnet, daß** 70 bis 90 Gew.-% des zuvor im Rückstand befindlichen Amins zurückgewonnen werden.
